(19) Europäisches Patentamt / European Patent Office / Office européen des brevets

(11) **EP 4 438 025 A1**

(12) **EUROPEAN PATENT APPLICATION**
published in accordance with Art. 153(4) EPC

(43) Date of publication:
**02.10.2024 Bulletin 2024/40**

(21) Application number: **22968567.2**

(22) Date of filing: **16.12.2022**

(51) International Patent Classification (IPC):
*A61K 6/802* (2020.01)     *A61C 5/77* (2017.01)
*A61C 13/083* (2006.01)     *A61K 6/818* (2020.01)
*C04B 35/111* (2006.01)     *C04B 35/486* (2006.01)

(52) Cooperative Patent Classification (CPC):
**A61C 5/77; A61C 13/083; A61K 6/802;**
**A61K 6/818; C04B 35/111; C04B 35/486**

(86) International application number:
**PCT/JP2022/046484**

(87) International publication number:
**WO 2024/127659 (20.06.2024 Gazette 2024/25)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Kuraray Noritake Dental Inc.**
**Kurashiki-shi, Okayama 710-0801 (JP)**

(72) Inventors:
• **NAKANO, Kirihiro**
**Miyoshi-shi, Aichi 470-0293 (JP)**

• **SAKAMOTO, Hiroyuki**
**Miyoshi-shi, Aichi 470-0293 (JP)**
• **KASHIKI, Nobusuke**
**Miyoshi-shi, Aichi 470-0293 (JP)**
• **KATO, Shinichiro**
**Miyoshi-shi, Aichi 470-0293 (JP)**
• **ISHINO, Hiroshige**
**Miyoshi-shi, Aichi 470-0293 (JP)**

(74) Representative: **D Young & Co LLP**
**3 Noble Street**
**London EC2V 7BQ (GB)**

(54) **OXIDE CERAMIC PARTIALLY SINTERED BODY FOR DENTAL USE WHICH HAS FAVORABLE POLISHING PROPERTIES, AND METHOD FOR PRODUCING SAME**

(57) The present invention provides a pre-sintered body of oxide ceramics that possesses excellent polishability and exhibits aesthetics due to high evenness on both the polished surface of the pre-sintered body and the surface of the sintered body after sintering. The invention also provides a method for producing such oxide ceramic pre-sintered bodies. The present invention relates to an oxide ceramic pre-sintered body for dental use comprising oxide ceramic particles with an average roundness of primary particles of 0.81 or more and having a relative density of 43 to 63%.

FIG.1

EP 4 438 025 A1

**Description**

TECHNICAL FIELD

The present invention relates to an oxide ceramic pre-sintered body for dental use comprising oxide ceramics and that can be desirably polished with tools, and to a method for producing such oxide ceramic pre-sintered bodies.

BACKGROUND ART

Over the last years, sintered bodies of oxide ceramics have become prevalent as dental materials. Such dental materials are used as sintered bodies with accurately processed dimensions and surfaces tailored to the clinically affected area of the patient. Processing to the desired shape is achieved by machining such as CAD/CAM.
Aluminum oxide (alumina) and zirconium oxide (zirconia) represent examples of oxide ceramics used as dental materials. Zirconia, in particular, has seen a rising demand due to its outstanding strength and relatively superior aesthetics, propelled by the decreasing price of this material in recent years.
However, zirconia sintered bodies involve productivity and cost-related issues, such as being too hard to machine, prone to cracking during machining, prolonged machining times, and a high frequency of processing tool replacements.
Because of these issues, production of zirconia sintered bodies generally involves machining a zirconia pre-sintered body in a semi-sintered state to prepare a cut or ground workpiece in a shape close to the desired shape resembling, for example, a tooth or a part of a tooth, instead of machining a zirconia sintered body. The cut or ground workpiece can then be fired at or above the sintering temperature to obtain a zirconia sintered body with the desired shape. The zirconia pre-sintered body is obtained by molding a raw material powder into a disc, cuboidal, or other such shapes, and firing (or "pre-sintering" as it is also referred to hereinbelow) the resultant molded body in a temperature range that does not cause sintering.
Concerning oxide ceramics other than zirconia, examples using alumina are proposed in, for example, Patent Literatures 1 to 3. Differing in refractive index from zirconia, alumina is more advantageous in terms of translucency after sintering. Additionally, because alumina has wide applications as sintered bodies except for porous bodies such as heat insulating materials, and there is no need to prepare pre-sintered bodies, it is commonly practiced to obtain sintered bodies through sintering of molded bodies.
In view of dental aesthetics, the sintered bodies of oxide ceramics produced are typically polished to provide surface smoothness.
However, due to the high hardness of sintered bodies, polishing requires a significant amount of time. Moreover, remanufacturing is necessary when the surface of sintered bodies breaks (chips) during polishing. There accordingly is room for improvement from the perspective of productivity and economy.

CITATION LIST

Patent Literature

Patent Literature 1: JP 2015-37537 A
Patent Literature 2: JP 2012-180275 A
Patent Literature 3: WO2009/045940

SUMMARY OF INVENTION

Technical Problem

Against this backdrop, the present inventors have found that by enabling polishing during the pre-sintering phase and achieving the desired surface properties (for example, evenness after polishing), it is possible to reduce both time and potential risks involved in polishing after sintering.
In order to improve the polishability of the pre-sintered body, one may consider decreasing the relative density of the pre-sintered body to reduce resistance during polishing. However, excessive reduction in relative density could render the pre-sintered body overly soft, leading to breakage, and/or diminish its sinterability, resulting in issues where the relative density fails to improve after sintering.
For example, Patent Literature 1 discloses a production method involving shaping and polishing bioactive alumina, describing surface polishing of alumina sintered bodies. However, the polishing of sintered bodies is time-consuming, with concerns arising around potential separation of some particles, consequently diminishing smoothness, instead of improving it. Moreover, as previously mentioned, alumina is extensively used as sintered bodies except for porous bodies such as heat insulating materials, and there is no need to prepare pre-sintered bodies. Consequently, Patent Literature 1 does not contemplate smoothing a pre-sintered body before sintering.

Patent Literature 2 describes a method for producing an alumina sintered body, whereby a molded body obtained by using an alumina powder with an average particle diameter of 0.2 to 1.0 $\mu$m is fired at 1,480 to 1,600°C. However, in Patent Literature 2, there is no indication of dental applications, and no consideration is given to pre-sintered bodies with high polishability. Moreover, Patent Literature 2 does not address particle roundness, and because of the large particle size, the pre-sintered body does not exhibit good polishability.

Patent Literature 3 describes hardness and porosity concerning abrasive grains suitable for polishing. However, it does not contemplate materials undergoing polishing. Even if the abrasive grain surface were regarded as a pre-sintered body, it cannot be said that this related art has identified the appropriate conditions to achieve smoothness for this pre-sintered body surface (abrasive grain surface).

Patent Literatures 1 to 3 do not address polishing and smoothing a pre-sintered body prior to sintering, and fail to provide a smooth sintered body in a cost-effective and convenient manner.

It is accordingly an object of the present invention to provide an oxide ceramic pre-sintered body that possesses excellent polishability and exhibits aesthetics due to high evenness on both the polished surface of the pre-sintered body and the surface of the sintered body after sintering. Another object of the invention is to provide a method for producing such oxide ceramic pre-sintered bodies.

Solution to Problem

The present inventors conducted intensive studies to find a solution to the foregoing issues, and found that an oxide ceramic pre-sintered body comprising oxide ceramic particles with an average roundness of primary particles of 0.81 or more and having a relative density of 43 to 63% exhibits high polishability. This led to the completion of the present invention after further examinations.

Specifically, the present invention includes the following.

[1] An oxide ceramic pre-sintered body for dental use comprising oxide ceramic particles with an average roundness of primary particles of 0.81 or more and having a relative density of 43 to 63%.

[2] The oxide ceramic pre-sintered body for dental use according to [1], wherein the particles have an average primary particle diameter of 30 to 600 nm.

[3] The oxide ceramic pre-sintered body for dental use according to [1] or [2], which has a three-point flexural strength of 10 to 50 MPa.

[4] The oxide ceramic pre-sintered body for dental use according to any one of [1] to [3], which has a surface roughness Ra of 1.70 $\mu$m or less after polishing.

[5] The oxide ceramic pre-sintered body for dental use according to any one of [1] to [4], which has a surface roughness Rz of 54 $\mu$m or less after polishing.

[6] The oxide ceramic pre-sintered body for dental use according to any one of [1] to [5], wherein the oxide ceramic particles comprise zirconia and/or alumina.

[7] The oxide ceramic pre-sintered body for dental use according to [6], wherein the alumina comprises $\alpha$-alumina particles with a purity of 99.5% or more.

[8] The oxide ceramic pre-sintered body for dental use according to [6] or [7], which further comprises a sintering aid, and the sintering aid comprises at least one element selected from the group consisting of a Group 2 element, Ce, Zr, and Y

[9] The oxide ceramic pre-sintered body for dental use according to any one of [1] to [8], which has a surface roughness Ra of 1.40 $\mu$m or less after being fired into a sintered body under atmospheric pressure without a hot isostatic pressing process.

[10] The oxide ceramic pre-sintered body for dental use according to any one of [1] to [9], which has a surface roughness Rz of 51 $\mu$m or less after being fired into a sintered body under atmospheric pressure without a hot isostatic pressing process.

[11] A method for producing an oxide ceramic pre-sintered body for dental use, comprising the steps of:

press molding an oxide ceramic composition at a surface pressure of 20 to 600 MPa; and
firing the resultant molded body at 400°C or more and less than 1,200°C under atmospheric pressure,
the oxide ceramic pre-sintered body for dental use comprising oxide ceramic particles with an average roundness of primary particles of 0.81 or more and having a relative density of 43 to 63%.

[12] The method for producing an oxide ceramic pre-sintered body for dental use according to [11], wherein the oxide ceramic particles comprise zirconia and/or alumina.

[13] The method for producing an oxide ceramic pre-sintered body for dental use according to [12], wherein the alumina comprises $\alpha$-alumina particles with a purity of 99.5% or more.

[14] The method for producing an oxide ceramic pre-sintered body for dental use according to any one of [11] to [13], wherein the oxide ceramic pre-sintered body for dental use further comprises a sintering aid, and the sintering aid comprises at least one element selected from the group consisting of a Group 2 element, Ce, Zr, and Y

[15] A method for producing an oxide ceramic sintered body for dental use, comprising the step of polishing an oxide ceramic pre-sintered body for dental use of any one of [1] to [10] with dental polishing equipment.

[16] The method for producing an oxide ceramic sintered body for dental use according to [15], which comprises the step of sintering the oxide ceramic pre-sintered body for dental use under atmospheric pressure without a hot isostatic pressing process.

Advantageous Effects of Invention

According to the present invention, an oxide ceramic pre-sintered body can be provided that possesses excellent polishability and exhibits aesthetics due to high evenness on both the polished surface of the pre-sintered body and the surface of the sintered body after sintering. The invention can also provide a method for producing such oxide ceramic pre-sintered bodies.

According to the present invention, the surface of a pre-sintered body that has been cut or ground by, for example, CAD/CAM can be polished with ease in surface processing of dental materials before sintering, providing an easy way to achieve a smooth surface. This has made it possible to provide a pre-sintered body possessing good smoothness, and a sintered body having good smoothness, along with methods of production of these.

Specifically, alumina exhibits high hardness after sintering, making it challenging to polish the surface after sintering. The present invention, however, has enabled the provision of a pre-sintered body that can be accurately and easily processed before sintering, along with a sintered body, and methods of production of these.

The present invention provides outstanding surface smoothness also in the sintered body, achieved through surface polishing of a pre-sintered body, for example, by polishing side surfaces, after it is processed into a crown shape.

The present invention can also provide a zirconia pre-sintered body having excellent machinability (cutting and grinding properties), and a method of production thereof.

BRIEF DESCRIPTION OF DRAWINGS

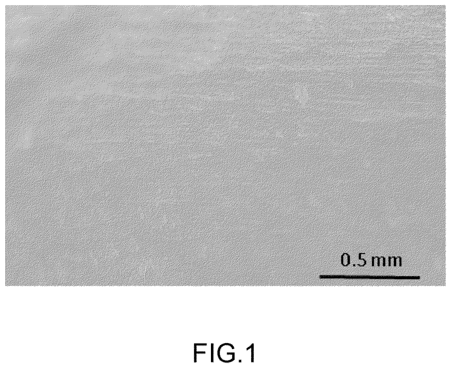

[FIG. 1] A light micrograph depicting a polished surface of a pre-sintered body according to Example 1.

DESCRIPTION OF EMBODIMENTS

An oxide ceramic pre-sintered body for dental use of the present invention has an average roundness of primary particles of 0.81 or more, and a relative density of 43 to 63%.

A pre-sintered body of the present invention is described below.

The pre-sintered body can be a precursor (intermediate product) of a sintered body.

In this specification, "pre-sintered body" refers to a state where oxide ceramic particles have formed necks, and have solidified without being fully sintered.

The pre-sintered body may have a predetermined shape (e.g., a block shape, for example, such as a disc shape or cuboidal shape; or a shape of a dental product, for example, such as a crown shape).

The pre-sintered body may be a workpiece that has been processed into, for example, a crown shape. The pre-sintered body will be referred to as "workpiece", or "cut or ground workpiece" when it has been processed.

The workpiece can be obtained by, for example, processing an oxide ceramic disc (pre-sintered body) into a dental product (for example, a crown-shaped prosthesis) with the CAD/CAM (Computer-Aided Design/Computer-Aided Manufacturing) system.

A pre-sintered body of the present invention comprises necks formed by particles of oxide ceramics (hereinafter, also referred to simply as "oxide ceramic particles"), and the polishability, machinability (cutting and grinding properties), and surface properties (evenness of the pre-sintered body and sintered body after polishing) vary according to the average roundness of these particles.

When the average roundness of the oxide ceramic particles contained in the pre-sintered body is 0.81 or more, it results in high polishability, and low surface roughness values Ra and/or Rz after polishing. Conversely, when the average roundness is less than 0.81, polishing the pre-sintered body leads to increased surface roughness values Ra and/or Rz, or the pre-sintered body becomes excessively hard, prolonging the working time.

The average roundness is preferably 0.82 or more, more preferably 0.83 or more, even more preferably 0.84 or more.

The method of measurement of average roundness is as described in the EXAMPLES section below.

In the present invention, surface roughness Ra means the arithmetic mean roughness Ra specified by JIS B 0601: 2013. Surface roughness Rz means the maximum height roughness Rz specified by JIS B 0601: 2013.

In an alumina pre-sintered body of the present invention, it is preferable to have a higher surface smoothness because it leads to enhanced aesthetic appeal after firing. The alumina pre-sintered body has a surface roughness Ra of preferably 1.70 $\mu$m or less, more preferably 1.65 $\mu$m or less, even more preferably 1.60 $\mu$m or less, particularly preferably 1.50 $\mu$m or less.

The alumina pre-sintered body has a surface roughness Rz of preferably 54 $\mu$m or less, more preferably 50 $\mu$m or less, even more preferably 45 $\mu$m or less, particularly preferably 35 $\mu$m or less.

The surface roughness Ra and Rz refer to measured values from polished alumina pre-sintered bodies.

By adjusting the average roundness, the present invention enables the production of an unpolished alumina pre-sintered body that shows a surface roughness Ra within the foregoing desired range (for example, 1.70 $\mu$m or less) after polishing, or an unpolished alumina pre-sintered body that shows a surface roughness Rz within the foregoing desired range (for example, 54 $\mu$m or less) after polishing.

The surface roughness of an alumina pre-sintered body of the present invention can be measured using known methods. Examples of such methods include the stylus method, and laser interferometry. Alternatively, the surface roughness may be measured by image analysis of a cross section after taking a cross-sectional image with a light microscope or electron microscope.

Preferably, the surface roughness Ra and Rz are measured values acquired by using the measurement method described in the EXAMPLES section below.

In a pre-sintered body of the present invention, the relative density can be controlled following the method of production described later.

A relative density of less than 43% means that the proportion of pores inside the pre-sintered body is high. This is not preferable because it reduces the number of particles in contact within the pre-sintered body, leading to excessive softness and susceptibility to breakage during polishing. Another reason is that it results in an increase in surface roughness Rz due to the sparse density within the pre-sintered body.

A relative density of more than 63% is not preferable because it leads to excessive hardness and prolonged working times. Another reason is that it causes chipping, resulting in an increase in surface roughness Ra and/or Rz.

Within the relative density range of 43 to 63%, the surface roughness Ra and Rz and hardness remain adequate during polishing of the pre-sintered body. Additionally, there is no increase in working time, and the polished surface of the pre-sintered body exhibits high evenness, and maintains high evenness even after sintering, enhancing the aesthetics of the sintered body. Moreover, the foregoing relative density range provides outstanding machinability (cutting and grinding properties), reducing the probability of chipping (hereinafter, also referred to as "chipping rate"), and maintaining a high level of evenness in the sintered body.

In view of even superior effectiveness of the present invention, the relative density is preferably 45 to 60%, more preferably 48% to 56%.

The relative density of the pre-sintered body can be calculated from the porosity of the pre-sintered body. Specifically, a mercury porosimeter can be used for the measurement and calculation of relative density.

The mercury porosimeter is preferably one capable of applying a mercury pressure of 15 to 30,000 psia, preferably 0.5 to 60,000 psia.

In view of reducing measurement errors, the preferred pressure resolution is 0.1 psia or higher.

Examples of the mercury porosimeter include AutoPore® IV 9500, manufactured by Micromeritics (USA).

The density of the pre-sintered body is defined as the density of a pre-sintered body obtained after granules resulting from drying of raw materials are filled into a specific mold (such as a die) and molded into a specific shape under pressure, and the resulting molded body is subsequently heated to remove organic components (such as binders) at a temperature that allows for removal of organic components, and heated again at a temperature that allows for moderate formation of a solid solution with yttria, and a moderate level of necking.

The temperature for the removal of organic components is not particularly limited, as long as it allows for removal of organic components such as binders. The temperature can be selected according to the type of binder or other organic components, and may be 150 to 500°C.

The temperature at which moderate neck formation occurs is preferably 400 or more and less than 1,200°C.

Details will be described later in conjunction with the firing temperature (hereinafter, also referred to as "pre-sintering temperature") in the pre-sintering step.

A pre-sintered body of the present invention comprises necks formed by particles of oxide ceramics (hereinafter, also referred to simply as "oxide ceramic particles"), and the extent of necking varies with the average particle diameter of these particles, resulting in a change in the hardness of the pre-sintered body.

The oxide ceramic particles contained in the pre-sintered body have an average primary particle diameter of preferably 30 to 600 nm, more preferably 40 to 580 nm, even more preferably 60 to 450 nm, most preferably 80 to 350 nm.

An average primary particle diameter of 600 nm or less in oxide ceramic particles is not preferable because the incorporation of smaller particles in the particle size distribution becomes less likely, reducing the occurrence of necking due to particle size differences, reducing the likelihood of hardness increase and preventing a prolonged working time. Another reason is that the localized absence of coarse particles reduces chipping, and enables a reduction of surface roughness Ra and/or Rz.

An average primary particle diameter of 30 nm or more is preferable because it does not lead to strong necking, and makes it less likely to cause an increase in hardness, preventing a prolonged working time, and reducing the surface roughness Ra and/or Rz.

The method of measurement of the average primary particle diameter in the pre-sintered body is as described in the EXAMPLES section below.

A pre-sintered body of the present invention internally comprises continuous holes (pores). The pores ensure space for particle movement when polishing tools come into contact with the pre-sintered body, reducing polishing resistance, enabling a reduction in the surface roughness Ra and/or Rz of the pre-sintered body.

In a pre-sintered body of the present invention, the amount of tool wear and chipping rate can be reduced when D10 is 10 nm or more and D90 is 90 nm or less in the cumulated pore distribution (cumulative distribution of pores).

In this specification, D10 and D90 refer to median pore diameters corresponding to the cumulative 10% and cumulative 90%, respectively, of pore diameters in a cumulative distribution of pores, starting from smaller values. The measurement of the cumulative distribution of pores, including D10 and D90, can be conducted using a method in compliance with JIS R 1655:2003.

With a D10 of 10 nm or more in a cumulative distribution of pores, the voids are not excessively small with respect to particles with an average primary particle diameter of 30 to 600 nm. That is, there is no excessive necking, enabling a reduction in the hardness of the pre-sintered body, and preventing a prolonged working time.

In view of a reduction of chipping rate and surface roughness, D10 is preferably 10 nm or more, more preferably 15 nm or more, even more preferably 25 nm or more.

With a D90 of 90 nm or less in a cumulative distribution of pores, it is possible to prevent coarseness in density or reduce the localized presence of coarse particles within the pre-sintered body with respect to particles having an average primary particle diameter of 30 to 600 nm, enabling a greater reduction in chipping rate during polishing, leading to even smaller surface roughness Ra and/or Rz.

In view of a reduction of chipping rate, D90 is preferably 79 nm or less, more preferably 66 nm or less, even more preferably 64 nm.

A pre-sintered body of the present invention exhibits a change in BET specific surface area with the average roundness of primary particle diameters of oxide ceramic particles within the pre-sintered body, as well as the relative density, average primary particle diameter, and pore distribution.

The BET specific surface area can be measured in compliance with JIS Z 8830:2013. For the measurement of BET specific surface area, commercially available products can be used, such as a full automatic specific surface area analyzer (manufactured by Mountech Co., Ltd. under the trade name Macsorb® HM Model-1200, BET flow method (single point/multi point)).

In view of reducing the amount of tool wear and chipping rate, a pre-sintered body of the present invention has a BET specific surface area of preferably 5 $m^2$/g or more, more preferably 7.5 $m^2$/g or more, even more preferably 8 $m^2$/g or more.

With a BET specific surface area of 5 $m^2$/g or more, the average primary particle diameter is not excessively large, allowing for the reduction of an increase in chipping rate, or the prevention of excessive necking, thereby reducing an increase in working time.

The BET specific surface area is preferably 25 $m^2$/g or less, more preferably 20 $m^2$/g or less, even more preferably 15 $m^2$/g or less.

With a BET specific surface area of 25 $m^2$/g or less, the average primary particle diameter is not excessively small, preventing the pre-sintered body from becoming overly hard. This facilitates a reduction in polishing time and/or chipping rate, or allows for a reduction of density coarseness by ensuring sufficient necking, making it easier to reduce the chipping rate.

In the present invention, "BET specific surface area" refers to the specific surface area measured without distinguishing between primary particles and secondary particles. Concerning the BET specific surface area of a pre-sintered body of the present invention and the BET specific surface area of the composition mentioned later, the extent of necking is preferably such that the numerical difference obtained by subtracting the BET specific surface area of the pre-sintered body from the BET specific surface area of the composition is within 10 $m^2$/g. This makes it possible to maintain good polishability.

The BET specific surface area of the pre-sintered body can be measured using known devices. The BET specific surface area can be measured in compliance with JIS Z 8830: 2013. For the measurement of BET specific surface area, commercially available products can be used, such as a full automatic specific surface area analyzer (manufactured by Mountech Co., Ltd. under the trade name Macsorb® HM Model-1200, BET flow method (single point/multi point)).

Concerning the polishability of a pre-sintered body of the present invention, the polishability is also influenced by the strength of the pre-sintered body. The strength of a pre-sintered body according to the present invention can be evaluated by, for example, measuring the flexural strength of the pre-sintered body. The three-point flexural strength of a pre-sintered body according to the present invention can be measured in compliance with JIS R 1601:2008.

In order to ensure strength that enables machining, the pre-sintered body has a three-point flexural strength of preferably 10 MPa or more, more preferably 18 MPa or more, even more preferably 20 MPa or more.

The pre-sintered body has a three-point flexural strength of preferably 10 MPa or more because it can reduce the possibility of the pre-sintered body breaking during polishing.

For easy polishing, the pre-sintered body has a three-point flexural strength of preferably 50 MPa or less, more preferably 45 MPa or less, even more preferably 40 MPa or less, particularly preferably 35 MPa or less.

In view of altering polishability and/or hardness, a pre-sintered body of the present invention has a Vickers hardness of preferably 350HV 5/30 or less, more preferably 300HV 5/30 or less, even more preferably 100HV 5/30 or less.

When the Vickers hardness is 350HV 5/30 or less, it demonstrates superior machinability (cutting and grinding properties), leading to reduced chipping rates and reduced increases in the amount of tool wear.

Here, "HV 5/30" means a Vickers hardness under the application of a load (test force) of 5 kgf for a duration of 30 seconds.

A pre-sintered body of the present invention can have reduced chipping rates with the Vickers hardness falling in the foregoing predetermined ranges. The Vickers hardness in the present invention is measured using a method in compliance with JIS Z 2244:2020.

The Vickers hardness can be measured as follows, for example.

An example method involves subjecting the pre-sintered body to a 5 kgf load for 30 seconds using a Falcon 500 manufactured by Innovatest, and calculating the HV value. For example, the HV value is calculated as a mean value for n = 10.

The oxide ceramic particles used in the present invention are not particularly limited, and include those containing, for example, zirconia, alumina, titania, silica, niobium oxide, tantalum oxide, and yttria. The oxide ceramics may be used alone, or two or more thereof may be used in combination. In view of enhancing the aesthetic qualities and strength of the sintered body as a dental material, the oxide ceramic particles are preferably those containing zirconia and/or alumina, more preferably those containing zirconia and/or alumina as main components.

The following describes embodiments where oxide ceramic particles contain alumina as a main component, while also discussing situations where the oxide ceramic is zirconia, as needed.

A composition containing alumina as a main component among the aforementioned oxide ceramics is preferable because of its ability to enhance the aesthetic qualities of the sintered body as a dental material, as well as exhibiting excellent chemical stability. Among variants of alumina, $\alpha$-alumina with a purity of 99.5% or more is more preferred for its low impurity content, and the ability to reduce the impurity-related formation of a glass phase at crystal grain boundaries, prevent the coarsening of crystal grains (grains), and inhibit a decrease of aesthetic qualities in the sintered body as a dental material.

Using $\alpha$-phase alumina ($\alpha$-alumina) as the starting raw material is preferable because, with its high corrosiveness and high stability at elevated temperatures, it enables uniform control of the pre-sintered body, and facilitates easier reduction of the amount of tool wear or chipping rate, in addition to enabling densification of grain size in the crystalline structure within the sintered body.

Considering these factors, it is particularly preferable that the alumina particles contained in a pre-sintered body of the present invention comprise $\alpha$-alumina particles with a purity of 99.5% or more.

The alumina raw material can be acquired using methods, for example, such as the alkoxide method, modified Bayer method, ammonium alum pyrolysis method, and ammonium dawsonite pyrolysis method, preferably the alkoxide method. The alkoxide method allows for enhancement of purity in a powder of alumina raw material, making it easier to achieve a uniform particle size distribution. As a specific example, aluminum hydroxide obtained through hydrolysis of purified aluminum alkoxide is fired in air at 1,100°C or higher temperatures.

Examples of the alumina raw material include $\alpha$-alumina with a purity 99.99% or more manufactured as the AA grade ($\alpha$-alumina), AKP grade ($\alpha$-alumina), or NXA grade (e.g., NXA-100, NXA-150) by Sumitomo Chemical Co., Ltd. (These are all ultrafine $\alpha$-alumina.)

A certain preferred embodiment of an oxide ceramic pre-sintered body of the present invention is, for example, an oxide ceramic pre-sintered body that comprises alumina or zirconia.

] In view of enhancing strength after sintering, and providing high aesthetic qualities in particular, it is preferable that an alumina pre-sintered body of the present invention further comprise a sintering aid (an auxiliary agent that accelerates and stabilizes sintering of alumina).

The sintering aid contained in an alumina pre-sintered body of the present invention preferably comprises at least one element selected from the group consisting of a Group 2 element (Be, Mg, Ca, Sr, Ba, Ra), Ce, Zr, and Y, more preferably at least one element selected from the group consisting of Mg, Ca, Sr, Ba, Ce, Zr, and Y, even more preferably at least one element selected from the group consisting of Mg, Ce, Zr, and Y

The sintering aid is most preferably a magnesium compound.

Examples of the magnesium compound include oxides, nitrates, acetates, hydroxides, and chlorides.

The magnesium compound is not limited, as long as it is a magnesium compound that turns into an oxide below 1,200°C during a sintering process in the atmosphere. Most preferred examples include magnesium nitrate, magnesium chloride, magnesium hydroxide, and magnesium acetate.

Examples of the sintering aid include $MgCl_2$, $Mg(OH)_2$, $CeO_2$, $ZrO_2$, and $Y_2O_3$.

Generally, the content of the sintering aid in a powder of alumina raw materials according to the present invention is preferably 10 ppm to 5,000 ppm, more preferably 20 ppm to 3,000 ppm, even more preferably 50 ppm to 1,500 ppm in terms of the aforementioned elements (for example, in terms of a Mg element). In this specification, ppm means ppm by mass.

When the content of the sintering aid (preferably, a magnesium compound) is low, it may result in a sintered body with a shade whiter than natural teeth, whereas an excessive content may impart an overly strong red tint. The mechanism by which the sintering aid enhances sintering density is believed to involve the exclusion of pores from the system by preventing the pores from being integrated into the grains, probably as a result of the sintering aid existing as a distinct phase at the grain boundaries, and inhibiting the growth and propagation of grain boundaries.

In applications requiring a sintered body with high purity, for example, 99.99 mass% or more, the content of the sintering aid in the alumina powder may be 10 to 100 ppm, or 20 to 50 ppm, in terms of the constituent element of the sintering aid (for example, in terms of a Mg element). The content of the sintering aid in an alumina pre-sintered body of the present invention and in the alumina composition mentioned later aligns with the content of the sintering aid in the alumina powder.

Another certain preferred embodiment is, for example, an oxide ceramic pre-sintered body for dental use comprising zirconia.

The main components of the oxide ceramics contained in a pre-sintered body of the present invention may be zirconia, and a stabilizing agent capable of preventing a phase transformation of zirconia (hereinafter, also referred to simply as "stabilizing agent"). The stabilizing agent is preferably one capable of forming partially-stabilized zirconia. Examples of the stabilizing agent include calcium oxide (CaO), magnesium oxide (MgO), yttria, cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide (PreOn, $Pr_2O_3$), samarium oxide ($Sm_2O_3$), europium oxide ($Eu_2O_3$), and thulium oxide ($Tm_2O_3$). Preferred is yttria.

In the case of a pre-sintered body comprising zirconia as the main component of the oxide ceramic (hereinafter, also referred to simply as "zirconia pre-sintered body"), it is preferable in a zirconia pre-sintered body of the present invention and a sintered body thereof that the content of the stabilizing agent (preferably, yttria) be 3.0 to 8.0 mol%, more preferably 3.2 to 6.5 mol%, even more preferably 3.5 to 6.0 mol%, particularly preferably 3.9 to 5.4 mol% relative to the total mole of zirconia and the stabilizing agent. When the content of stabilizing agent is less than 3.0 mol%, it leads to a zirconia sintered body with insufficient translucency. When the content of stabilizing agent is more than 8.0 mol%, it leads to an elevated presence of a phase transforming into the tetragonal and/or cubic crystal systems, resulting in an increased chipping rate and reduced polishability, and a strength reduction in the zirconia sintered body.

The content of the sintering aid or stabilizing agent in a pre-sintered body of the present invention and in a sintered body thereof can be measured by, for example, inductively coupled plasma (ICP) emission spectral analysis, X-ray fluorescence analysis (XRF), scanning or transmission electron microscopy (SEM or TEM), energy dispersive X-ray analysis or wavelength dispersive X-ray analysis (EDX or WDX), or field emission electron probe microanalysis (FE-EPMA).

In dental products, surface smoothness is important in terms of aesthetics, and it is preferable to have a low chipping rate in the pre-sintered body. A low chipping rate is also preferred in terms of reducing the effort needed for reworking the cut or ground workpiece as a dental material after firing. The chipping rate is preferably 10% or less, more preferably 7% or less, even more preferably 3% or less.

The method of measurement of chipping rate is as follows.

A light microscope is used to acquire a side-surface image of a 1 mm thick disc machined for the measurement of tool wear. The chipped region is rendered black, distinguishing it from the white area outside the black area (binarized). The chipping rate can be calculated as a percentage of the black area relative to the combined black and white areas. Image analysis software (manufactured by Hakuto Co., Ltd. under the trade name Image-Pro Plus) can be used for the area measurement.

Alumina and sintering aids may be used with zirconium oxide and stabilizing agents, provided that these are combined in small amounts without diminishing polishability and aesthetics after sintering.

For example, the mole ratio of zirconium oxide with respect to alumina ($ZrO_2/Al_2O_3$) is preferably 99.99 to 98, more preferably 99.9 to 99.

The mole ratio of zirconium oxide with respect to alumina ($ZrO_2/Al_2O_3$) is preferably 0.01 to 2, more preferably 0.1 to 1.

The following describes an oxide ceramic composition for producing an oxide ceramic pre-sintered body of the present invention, taking an alumina composition as an example where the oxide ceramic is aluminum oxide. The term "alumina composition" can be read as referring to "oxide ceramic composition", except where specified otherwise. Similarly, should zirconium oxide be chosen as the oxide ceramic, it can be implemented as a zirconia composition in the same manner as the alumina composition, except where specified otherwise.

The alumina composition becomes a precursor of an alumina pre-sintered body of the present invention.

In this specification, the alumina composition and molded body refer to a state before firing, and involve no necking between alumina particles.

The content of alumina and sintering aids in an alumina composition of the present invention is calculated from their content in a predetermined alumina pre-sintered body, and is the same for the alumina composition and alumina pre-sintered body.

The form of the alumina composition is not limited, and an alumina composition of the present invention may have various forms, including a powder, a fluid with a powder added to solvent, and a molded body of a powder formed into a predetermined shape. When an alumina composition of the present invention has a powder form, it may be a cluster of granules. The granules are formed by the aggregation of primary particles.

In this specification, "primary particle" refers to a bulk representing the smallest unit. For example, "primary particle" refers to particles that appear spherical under an electron microscope (for example, a scanning electron microscope). The primary particles include alumina particles. When employing particulate sintering aids, the primary particles include particles of sintering aid, as well as alumina particles.

Preferably, the particles constituting the granules formed of the alumina composition are predominantly primary particles. Aggregates of primary particles are referred to as secondary particles. Preferably, the number of primary particles is greater than the number of secondary particles in visual confirmation through, for example, an electron microscope. Secondary particles are typically irregular in shape, and an increase in their quantity leads to sparse density during the press molding process described later, resulting in coarseness in the pre-sintered body. This leads to increased chipping during polishing of the pre-sintered body, and increased surface roughness Ra and/or Rz after polishing the pre-sintered body.

The average primary particle diameter of primary particles within the particles constituting the granules formed of the alumina composition influences the extent of necking during pre-sintering, and affects the hardness of the pre-sintered body.

Particles with an average primary particle diameter of 30 nm or more are preferable because it does not result in a decrease in the surface area of primary particles within the pre-sintered body, preventing overly strong necking and reducing a hardness increase.

Particles with an average primary particle diameter of 600 nm or less are preferable because the incorporation of smaller particles in the particle size distribution becomes less likely, reducing the localized occurrence of necking due to particle size differences, thereby reducing coarseness.

The average primary particle diameter is preferably 30 to 600 nm, more preferably 40 to 580 nm, even more preferably 60 to 450 nm, most preferably 80 to 350 nm.

The primary particles within the particles constituting the granules formed of the alumina composition may be a combination of two types of alumina particles having different average primary particle diameters. An example is a combination of NXA-100 and NXA-150, when, for example, NXA is employed as primary particles of alumina particles constituting the granules and satisfying the foregoing average primary particle diameter range specified for the primary particles within the particles constituting the granules. These may be freely combined, as long as the average roundness and relative density of the oxide ceramic particles in the pre-sintered body are satisfied, more preferably by satisfying the average primary particle diameter, the strength of the pre-sintered body, and the cumulative distribution of pores.

The particles constituting the granules formed of the alumina composition has a BET specific surface area of preferably 5 $m^2/g$ or more, more preferably 7.5 $m^2/g$ or more, even more preferably 8 $m^2/g$ or more, as measured in compliance with JIS Z 8830:2013.

With a BET specific surface area of 5 $m^2/g$ or more, it is easier to lower the sinterable temperature. This facilitates easier sintering, or makes it easier to inhibit the reduction in translucency due to the opacification occurring in the sintered body after sintering.

The BET specific surface area is preferably 25 $m^2/g$ or less, more preferably 20 $m^2/g$ or less, even more preferably 15 $m^2/g$ or less.

With a BET specific surface area of 25 $m^2/g$ or less, the average primary particle diameter is not excessively small, preventing the pre-sintered body from becoming overly hard. This facilitates a reduction in the amount of polishing time and/or chipping rate during polishing, allowing for a further reduction in surface roughness Ra and/or Rz, or a reduction of density coarseness by ensuring sufficient necking, enabling a greater reduction in chipping rate during the polishing of the pre-sintered body, and a further reduction in surface roughness Ra and/or Rz.

An alumina composition of the present invention may employ a granular form in 50% or more, preferably 70% or more, more preferably 80% or more, even more preferably 90% or more of the alumina in the alumina composition.

When an alumina composition of the present invention does not employ a granular form, the alumina particles constituting the powder may have the average particle diameter and the BET specific surface area noted above.

The average particle diameter of granules in an alumina composition of the present invention (secondary particle diameter; hereinafter, also referred to as "average granule diameter") is preferably 10 $\mu$m or more, more preferably 12 $\mu$m or more, even more preferably 14 $\mu$m or more. When the average granule diameter is less than 10 $\mu$m, there is a risk of trapping air in the process of placing the granules in a die, leading to inadequate degassing during molding. This may result in the inability to fabricate a uniform and dense molded body. Additionally, there is a risk of granules being ejected through gaps during molding, potentially resulting in a molded body that does not meet the predetermined quantitative requirements. The average granule diameter is preferably 200 $\mu$m or less, more preferably 190 $\mu$m or less, even more preferably 180 $\mu$m or less, particularly preferably 150 $\mu$m or less, most preferably 100 $\mu$m or less. When the average granule diameter exceeds 200 $\mu$m, there is an increased likelihood of cavity formation within the granules. Additionally, voids are more likely to occur when placing the granules in a die. These phenomena pose a risk of inadequate degassing during molding, potentially resulting in the inability to fabricate a dense molded body. There is also a risk of increased shrinkage during molding, potentially leading to the inability to fabricate a molded body of the desired size. It is preferable that 50% or more of the alumina in the alumina composition constitute granules. Preferably, the average granule diameter is measured using a method that does not disrupt the granules. The average granule diameter can be measured using a method, for example, such as dry sieving or wet sieving.

Measurement by dry sieving can be conducted following the test sieving described in JIS Z 8815:1994. Both manual sieving and mechanical sieving are applicable; however, mechanical sieving is preferred.

For sieving, the test sieve described in JIS Z 8801-1:2019 can be used.

Ro-Tap sieve shakers or sonic sieving analyzers may be used as measurement instruments for sieving, for example. Examples of the Ro-Tap sieve shakers include RPS-105M manufactured by Seishin Enterprise Co., Ltd. Examples of the sonic sieving analyzers include Robot Sifter RPS-01 and Robot Sifter RPS-02 manufactured by Seishin Enterprise Co., Ltd.

It is preferable that granules in an alumina composition of the present invention have high sphericity. By increasing the sphericity of granules, it is possible to promote mixing at the interface between layers when stacking alumina powders of different compositions.

With increased sphericity, it is also possible to increase the packing density of when an alumina powder is filled into a mold to prepare a molded body, even when the average particle diameter is the same. The strength and translucency of the sintered body can increase by increasing the packing density, which is a density of a molded body formed into a specific shape under pressure after alumina granules are filled into a specific mold (such as a die).

It is also possible to more easily fill granules into angular portions when the mold has angular portions.

The sphericity of granules in an alumina composition of the present invention can be represented by parameters, for example, such as light bulk density, and heavy bulk density.

In view of increasing granule flow (ease of loading) to reduce coarseness in the density of the molded body obtained, an alumina composition of the present invention has a light bulk density of preferably 0.6 g/cm$^3$ or more, more preferably 0.7 g/cm$^3$ or more, even more preferably 0.8 g/cm$^3$ or more, particularly preferably 0.9 g/cm$^3$ or more.

The light bulk density can be measured in compliance with JIS R 9301-2-3:1999.

In view of increasing granule flow (ease of loading) to reduce coarseness in the density of the molded body obtained, an alumina composition of the present invention has a heavy bulk density of preferably 0.8 g/cm$^3$ or more, more preferably 0.9 g/cm$^3$ or more, even more preferably 1.0 g/cm$^3$ or more.

The heavy bulk density can be measured in compliance with JIS R 9301-2-3:1999.

An alumina composition of the present invention preferably comprises a binder.

The binder may be, for example, an organic binder. Examples of the organic binder include common binders such as acrylic binders, acrylate binders, paraffinic binders, fatty acid binders, and polyvinyl alcohol binders. Preferred among these organic binders is one having a carboxyl group in the molecular chain, or a derivative of carboxylic acids, more preferably an acrylic binder, even more preferably a polyacrylate having water solubility. The polyacrylate may be a product of copolymerization of acrylic acid or methacrylic acid with maleic acid, and may contain sulfonic acid. Examples of the cations of the salt include sodium and ammonium.

Depending on the content of the binder in an alumina composition of the present invention, it is possible to adjust the distance between primary particles in the alumina composition, and the cumulative distribution of pores, making it easier to adjust the Vickers hardness or the strength of the pre-sintered body by increasing or decreasing these factors.

The binder content is preferably 1.2 to 2.8 mass%, more preferably 1.5 to 2.5 mass%, even more preferably 1.8 to 2.2 mass% in the whole alumina composition. When the binder content is 1.2 mass% or more in the whole alumina composition, the pre-sintered body does not become overly strong, eliminating the risk of hardening when removing the cut workpiece.

When the binder content is 2.8 mass% or less, the strength of the pre-sintered body does not overly decrease, reducing the possibility of the workpiece detaching during cutting, in addition to facilitating a reduction in chipping rate.

An alumina composition of the present invention may optionally comprise additives other than sintering aids (additives excluding $CeO_2$, $ZrO_2$, and $Y_2O_3$), such as colorants (including pigments, composite pigments, and fluorescent agents), titanium oxide ($TiO_2$), silica ($SiO_2$), dispersants, and antifoaming agents. These components may be used alone, or two or more thereof may be used as a mixture.

Examples of the pigments include oxides of at least one element selected from the group consisting of Ti, V, Cr, Mn, Fe, Co, Ni, Zn, Y, Zr, Sn, Sb, Bi, Ce, Sm, Eu, Gd, and Er.

Examples of the composite pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$.

Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

The additives may be added during mixing or pulverization, or may be added after pulverization.

A certain embodiment is, for example, an oxide ceramic pre-sintered body for dental use that has a surface roughness Ra of 1.40 $\mu$m or less after being fired into a sintered body under atmospheric pressure without a hot isostatic pressing process (HIP).

Another certain embodiment is, for example, an oxide ceramic pre-sintered body for dental use that has a surface roughness Rz of 51.0 $\mu$m or less after being fired into a sintered body under atmospheric pressure without a hot isostatic pressing process.

Yet another certain embodiment is, for example, an oxide ceramic pre-sintered body for dental use that has an average crystal grain size of 0.3 to 8.0 $\mu$m after being fired into a sintered body under atmospheric pressure without a hot isostatic pressing process. The average crystal grain size shares the same measurement method and preferred ranges as the average crystal grain size of the alumina sintered body described later.

A certain embodiment is a method for producing an oxide ceramic pre-sintered body for dental use, comprising the steps of:

> press molding an oxide ceramic composition at a surface pressure of 20 to 600 MPa; and
> firing the resultant molded body at 400°C or more and less than 1,200°C under atmospheric pressure,
> the oxide ceramic pre-sintered body for dental use comprising oxide ceramic particles with an average roundness of primary particles of 0.81 or more and having a relative density of 43 to 63%.

Another certain preferred embodiment is, for example, a method for producing an oxide ceramic pre-sintered body for dental use in which the oxide ceramic particles comprise zirconia and/or alumina in the above method of producing an oxide ceramic pre-sintered body for dental use. Zirconia and alumina are as described for the pre-sintered body.

The following descriptions of an oxide ceramic pre-sintered body producing method of the present invention are given through the case of a method of producing an alumina pre-sintered body, taking aluminum oxide as an example of the oxide ceramic.

The method can be implemented as a method of producing a zirconia pre-sintered body in the same fashion when the oxide ceramic is zirconium oxide, except where specified otherwise.

An example of an alumina pre-sintered body producing method comprises the steps of:

> producing an alumina composition comprising alumina particles and a sintering aid; and
> firing (pre-sintering) the alumina composition (for example, a molded body) to obtain an alumina pre-sintered body having an average roundness of primary particles of 0.81 or more and a relative density of 43 to 63%.

The content of the sintering aid is preferably 10 to 5,000 ppm.

The process of producing an alumina composition of the present invention is described first.

First, alumina and a sintering aid are mixed in predetermined proportions to prepare a mixture (mixing step). For example, when the sintering aid is magnesium chloride, alumina and magnesium chloride may be mixed at a mixing ratio that provides the foregoing contents. The mixing process may be dry mixing or wet mixing. In view of enabling the adjustment of the average roundness and relative density as desired in producing the pre-sintered body, the alumina composition may be pulverized (preferably, crushed) until the foregoing average primary particle diameter is achieved (pulverization step).

The mixing and pulverization may be performed in a single step. Pulverization can be performed by using, for example, a ball mill or bead mill after dispersing the composition and binder in a solvent such as water or alcohol (dispersing step). In view of enabling the adjustment of the average roundness and relative density as desired in producing the pre-sintered body, the composition is pulverized (preferably, crushed) to achieve an average primary particle diameter of, for example, 0.05 $\mu$m to 0.6 $\mu$m in the composition. For particle size adjustment, the composition may optionally be subjected to other processes (classification, elutriation).

The average primary particle diameter can be measured by laser diffraction/scattering particle size distribution analysis. For example, the average primary particle diameter can be measured by volume with ultrasonic waves being applied after a slurry diluted with water is subjected to 30 minutes of ultrasonication, using a laser diffraction/scattering particle size distribution analyzer (manufactured by Horiba Ltd. under the trade name Partica LA-950). After the mixing and/or pulverization step, the mixture may be dried by spray drying with a spray dryer or the like to achieve the aforementioned granular form in the alumina composition (drying step).

In the pulverization step, the average primary particle diameter of the alumina composition is preferably 30 to 600 nm, more preferably 40 to 580 nm, even more preferably 60 to 450 nm, particularly preferably 80 to 350 nm. When the average particle diameter of the alumina composition is 30 to 600 nm, it is possible to satisfy both surface roughness and hardness in the pre-sintered body after polishing.

Alumina and sintering aids may be separately prepared. For example, it is possible to independently provide separate preparation steps (for example, production steps) for alumina and sintering aids, instead of simultaneously precipitating alumina and sintering aids (in the same step). In this way, the aforementioned $\alpha$-alumina can be obtained with high purity and a small primary particle diameter.

In the production of alumina compositions, sintering aids may be reacted with alumina through heat treatment, and the resulting product may be used for the pulverization and drying steps.

This enables the production of granules formed of the alumina composition, which serves as the raw material of alumina pre-sintered bodies.

The granule or powder can be formed into a molded body by applying an external force. The molding method is not limited to specific methods, and a suitable method may be appropriately selected according to intended use. For example, molding may be achieved by press molding, injection molding, stereolithography, slip casting, gel casting, filtration-casting, or casting. Molding may be performed in multiple stages. For example, the alumina composition may be subjected to a CIP process after press molding, or press molding and CIP molding may be repeated.

Examples of the press molding method include a uniaxial pressing process (hereinafter, also referred to as "uniaxial pressing"), a biaxial pressing process, and a CIP (Cold Isostatic Pressing) process. These may be performed in combination, as needed.

A molded body of the present invention may have a disc shape, a cuboidal shape, or a shape of a dental product (for example, a crown shape).

In certain embodiments, the press molding may be conducted by uniaxial pressing. The pressure molding step may yield, for example, a columnar molded body packed by uniaxially pressing alumina granules filled into a die. The molded body can increase its density with higher surface pressure during press molding. This makes it possible to increase the relative density of the resulting alumina pre-sintered body, and adjust the average roundness. Conversely, a hard alumina pre-sintered body results when the density of the molded body is excessively high, failing to achieve good machinability. In view of the ease of adjusting the average roundness and relative density of the alumina pre-sintered body to the desired ranges together with factors such as the average primary particle diameter of primary particles constituting the granules formed of the raw material alumina composition, and the pre-sintering temperature, the surface pressure in press molding is preferably 20 to 600 MPa, more preferably 25 to 400 MPa, even more preferably 30 to 200 MPa. When the surface pressure in pressing molding (for example, uniaxial pressing) is 20 MPa or more, the molded body exhibits superior shape retention. With a surface pressure of 600 MPa or less, it is possible to prevent excessive density increase in the molded body, making it easier to avoid hardening.

The preferred range of surface pressures in press molding may be 50 MPa or more, 80 MPa or more, 100 MPa or more, or 150 MPa or more, depending on factors such as the desired average roundness and relative density.

In certain embodiments, the surface pressure may be, for example, 20 to 200 MPa, 25 to 190 MPa, or 30 to 180 MPa, depending on factors such as the desired average roundness and relative density.

A molded body of the present invention encompasses molded bodies compacted by a high-temperature pressing process such as a CIP (Cold Isostatic Pressing) process. In view of the above, the hydraulic pressure is preferably 50 to 1,000 MPa, more preferably 100 to 600 MPa, even more preferably 150 to 300 MPa.

An alumina pre-sintered body according to the present invention becomes a precursor of the alumina sintered body described later. An alumina pre-sintered body according to the present invention includes those that have been subjected to a molding process. For example, an alumina pre-sintered body according to the present invention includes dental products (for example, crown-shaped prostheses) processed from pre-sintered alumina discs with the CAD/CAM (Computer-Aided Design/Computer-Aided Manufacturing) system.

The content of the alumina and sintering aids in an alumina pre-sintered body of the present invention is the same as that in the alumina composition to be fabricated into an alumina pre-sintered body. In view of the strength and translucency of sintered bodies fabricated from the pre-sintered bodies described below, it is preferable that the sintering aid in an alumina pre-sintered body of the present invention be uniformly dispersed as a magnesium compound.

The following describes the step of firing (pre-sintering) the molded body under atmospheric pressure (pre-sintering step).

The pre-sintering temperature in the pre-sintering step influences the average roundness of oxide ceramic particles contained in the pre-sintered body, and affects the Vickers hardness, or the strength of the pre-sintered body.

The pre-sintered body undergoes changes in polishability and hardness with the pre-sintering temperature.

The pre-sintering temperature (highest pre-sintering temperature) in an alumina pre-sintered body producing method of the present invention is preferably 400°C or more and less than 1,200°C. This is because, by maintianing the pre-sintering temperature in a temperature range where sintering does not take place, the particle surface can retain its shape (spherical shape) in the resulting structure. This ensures that the pre-sintered body does not become excessively hard, allowing for the maintenance of surface roughness and hardness during polishing.

It is preferable that pre-sintering increases the average roundness of oxide ceramic particles during the pre-sintering process without causing excessive necking. In view of this, the pre-sintering temperature is preferably a temperature that yields an average roundness of 0.81 or more for the oxide ceramic particles contained in the pre-sintered body.

When the oxide ceramic particles contained in the alumina composition (for example, a molded body) have small average primary particle diameters (for example, 30 nm to 150 nm), necking occurs at lower pre-sintering temperatures, increasing the Vickers hardness, or the strength of the pre-sintered body. For example, the pre-sintering temperature is preferably around 750°C (700 to 850°C) when the average primary particle diameter of the oxide ceramic particles contained in the alumina composition (for example, a molded body) is about 95 nm.

When the oxide ceramic particles contained in the alumina composition have small average primary particle diameters, a pre-sintering temperature of 1,200°C or more is not preferable because the average roundness decreases due to the formation of necks between particles, resulting in excessive hardness and a prolonged polishing time.

When the oxide ceramic particles contained in the alumina composition (for example, a molded body) have large average primary particle diameters (for example, 380 nm to 600 nm), necking does not occur at lower pre-sintering temperatures but takes place at higher pre-sintering temperatures, increasing the Vickers hardness, or the strength of the pre-sintered body. For example, the pre-sintering temperature is preferably around 1,150°C (1,100 or more and less than 1,200°C) when the average primary particle diameter of the oxide ceramic particles contained in the alumina composition (for example, a molded body) is about 580 nm.

When the oxide ceramic particles contained in the alumina composition (for example, a molded body) have large average primary particle diameters, a pre-sintering temperature of 1,000°C or less is not preferable because the average roundness does not increase, and necking between particles does not proceed, failing to increase the strength of the pre-sintered body or Vickers hardness, causing chipping during polishing, and a reduction of surface roughness Ra and/or Rz.

The average primary particle diameter of primary particles constituting the granules formed of the raw material oxide ceramic composition (for example, an alumina composition) is not necessarily the same as the average primary particle diameter of primary particles of the oxide ceramic particles (for example, alumina particles) contained in the pre-sintered body. However, the average primary particle diameter of primary particles of the oxide ceramic particles (for example, alumina particles) contained in the pre-sintered body can be adjusted to the desired range by adjusting the extent of necking through predetermined conditions that include the average primary particle diameter of primary particles constituting the granules formed of the raw material oxide ceramic composition (for example, an alumina composition), and the pre-sintering temperature, as noted above.

In view of adjusting the average roundness of primary particles of the oxide ceramic particles in the pre-sintered body to the desired range by controlling the extent of necking taking into consideration the relationship between the pre-sintering temperature and the average particle diameter of primary particles constituting the granules formed of the raw material oxide ceramic composition or alumina composition, the pre-sintering temperature is preferably a temperature that yields an average roundness of 0.81 or more for the oxide ceramic particles contained in the pre-sintered body, and a relative density of 43 to 63% for the pre-sintered body.

The pre-sintering temperature is preferably 600°C or more and less than 1,200°C, more preferably 750°C or more and 1,150°C or less.

It is preferable to maintain the highest pre-sintering temperature for a certain time period because it confines the hardness of the pre-sintered body within the preferred range, and may lead to a reduction in chipping rate.

The pre-sintering conditions depend on the average primary particle diameter of the pre-sintered body, and the density of the pre-sintered body. The holding time at the highest pre-sintering temperature is preferably 30 minutes to 6 hours. Preferably, the rate of temperature increase to the highest pre-sintering temperature, and the rate of temperature decrease from the highest pre-sintering temperature are 300°C/min or less.

An alumina pre-sintered body of the present invention can be machined to fabricate a workpiece. The processing method is not limited to specific methods, and a suitable method may be appropriately selected according to intended use. For example, an alumina disc (pre-sintered body) can be milled or ground into a shape of a dental product (for example, a crown-shaped prosthesis) with a CAD/CAM system to fabricate a workpiece.

The processing machine used for machining a pre-sintered body of the present invention is not particularly limited. For example, milling or grinding machines such as desktop processing machines and large machining centers (general-purpose processing machine) may be used, depending on the workpiece to be processed. Examples of such milling machines include tabletop processing machines, for example, such as DWX-50, DWX-4, DWX-4W, DWX-52D, and DWX-52DCi (manufactured by Roland DG Corporation). Alternatively, a grinding process may be employed.

The tools employed in the processing machines for machining a pre-sintered body of the present invention are not particularly limited. Milling burs and grinding burs recommended by processing machine suppliers can be suitably used. A KATANA® drill represents an example of milling burs used for milling machines.

The workpiece derived from an alumina pre-sintered body of the present invention displays level differences on its surface according to the machining process. If sintered with these level differences, the resulting surface of the sintered body retains irregularities corresponding to the level differences, necessitating polishing before its application as a dental material. It is therefore preferable that the surface of the workpiece derived from the pre-sintered body and having level differences be smoothed through cutting, grinding, or polishing.

The surface smoothness of the workpiece derived from an alumina pre-sintered body of the present invention can be improved using dental tools. The dental tools may be, for example, kits including burs and polishers, suitable for shape modification through cutting or grinding of dental ceramics, including porcelain, and improvement of surface properties through polishing. Examples of tools include Noritake Pro-Tec Diamond Point, carbide burs, Meister Cones, rubber points, and felt wheels manufactured by Kuraray Noritake Dental Inc.; TWIST DIA, Coarse, Medium, and Fine; and Cerapika for HP, a point for ceramics manufactured by P.D.R. (disc type for semi-polishing, teardrop type for semi-polishing, disc type for final polishing, and teardrop type for final polishing).

The surface smoothness of the workpiece derived from an alumina pre-sintered body of the present invention may be improved using tools. A polishing powder may be used when using tools to polish the workpiece derived from an alumina pre-sintered body of the present invention. Examples of such polishing powders include the Pearl Surface® C and Pearl Surface® F manufactured by Kuraray Noritake Dental Inc. When using abrasives, it is preferable to wash the workpiece because abrasives become foreign substances in sintering.

When using a tool to polish the workpiece derived from an alumina pre-sintered body of the present invention, the rotational speed of the tool is preferably 1,000 to 7,000 rpm. Lower speeds, below 1,000 ppm, not only prolong the process but also lead to hand instability due to recoil when the rotating tool collides with the pre-sintered body, resulting in difficulty achieving a smooth surface. Higher rotational speeds, exceeding 7,000 rpm, increase the tool's polishing force, resulting in excessive polishing of the pre-sintered body and difficulty achieving the desired shape. The rotational speed is more preferably 2,000 to 6,000 rpm, even more preferably 3,000 to 5,000 rpm.

The sintering step takes place after the workpiece derived from an alumina pre-sintered body of the present invention is polished with a tool. Here, it is preferable to remove any debris from the surface of the workpiece because debris may affect the shape and appearance after sintering when present on the surface. Preferably, debris is removed from the workpiece after final polishing, using, for example, a paining brush, until the absence of debris is visually confirmed.

The following descriptions of an oxide ceramic sintered body producing method of the present invention are given through the case of a method of producing an alumina sintered body, taking aluminum oxide as an example of the oxide ceramic.

An alumina sintered body of the present invention can be fabricated by sintering an alumina pre-sintered body of the present invention, or a cut or ground workpiece thereof, at a temperature that sinters the alumina particles (sintering step). When the average primary particle diameter is approximately 100 nm, the sinterable temperature (for example, highest sintering temperature) is, for example, preferably 1,300°C or more, more preferably 1,350°C or more, even more preferably 1,375°C or more.

The sinterable temperature is, for example, preferably 1,500°C or less, more preferably 1,450°C or less. The rate of temperature increase to the sinterable temperature, and the rate of temperature decrease from the sinterable temperature are preferably 300°C/min or less.

In the sintering step, the holding time at the sinterable temperature (for example, highest sintering temperature) is preferably 120 minutes or less, more preferably 90 minutes or less, even more preferably 75 minutes or less, yet more preferably 60 minutes or less, particularly preferably 45 minutes or less, most preferably 30 minutes or less. The holding time is preferably 1 minute or more, more preferably 3 minutes or more, even more preferably 5 minutes or more.

An alumina composition and alumina pre-sintered body of the present invention enable a shorter sintering process to be employed for the fabrication of a sintered body, without causing a decrease in the translucency and strength of the alumina sintered body fabricated. Particularly, it is possible to shorten the holding time at the highest sintering temperature for the fabrication of a sintered body (short sintering). This enhances production efficiency, and when an alumina pre-sintered body of the present invention is applied to dental products, the patient can experience less time-related stress because it takes a shorter time before a treatment can be started with a dental product after its dimensions are determined and the product is cut or ground for the treatment. It is also possible to reduce the energy cost.

In the sintering step, the holding time at the sinterable temperature (for example, highest sintering temperature) may be, for example, 25 minutes or less, 20 minutes or less, or 15 minutes or less.

Preferably, the rate of temperature increase and the rate of temperature decrease in the sintering process are set so as to reduce the time required for the sintering process. For example, the rate of temperature increase may be set so that the highest sintering temperature is reached in the shortest possible time, depending on the capabilities of the furnace. The rate of temperature increase to the highest sintering temperature may be, for example, 10°C/min or more, 50°C/min or more, 100°C/min or more, 120°C/min or more, 150°C/min or more, or 200°C/min or more. Preferably, the rate of temperature decrease is set to a rate that does not cause deformation due to shrinkage rate differences, or defects such as cracks, in the sintered body. For example, the sintered body may be allowed to cool at room temperature after the heating is finished.

A certain embodiment is, for example, a method for producing an oxide ceramic sintered body for dental use, comprising the step of polishing any of the aforementioned oxide ceramic pre-sintered bodies for dental use with dental polishing equipment. The dental polishing equipment is not particularly limited, and may be a known commercially available product. The polishing conditions are not particularly limited.

Another certain embodiment is, for example, a method for producing an oxide ceramic sintered body for dental use, comprising the step of sintering the oxide ceramic pre-sintered body for dental use under atmospheric pressure without a hot isostatic pressing process. In a method for producing an oxide ceramic sintered body for dental use according to the present invention, there is no need for a hot isostatic pressing (HIP) process. This eliminates the need for specialized equipment, allowing for easy production of an oxide ceramic sintered body for dental use.

The following descriptions of the oxide ceramic sintered body are given through the case of an alumina sintered body, taking aluminum oxide as an example of the oxide ceramic.

The alumina sintered body is obtained by sintering the alumina pre-sintered body or a workpiece thereof. Here, "alumina sintered body" refers to a state where alumina particles (powder) have sintered.

The alumina sintered body has a relative density of preferably 99.5% or more.

The relative density in the sintered body can be calculated as a ratio of the actual density, measured by the Archimedes method, with respect to the theoretical density. By "relative density", it means a value obtained by theoretically dividing density d1 by density d2, where d1 is the density of a sintered body after high-temperature firing of a molded body prepared by filling granules into a specific mold, and pressing the granules into a specific shape, and d2 is the density of alumina (with no internal voids).

An alumina sintered body of the present invention encompasses not only sintered bodies of molded alumina particles sintered under ordinary pressure and no applied pressure, but also sintered bodies compacted by a high-temperature pressing process such as a HIP (Hot Isostatic Pressing) process.

A greater relative density in an alumina sintered body of the present invention leads to fewer internal voids and less scattering of light. Because this results in an alumina sintered body with increased translucency ($\Delta L$), total transmittance, and linear light transmittance, and in view of improved aesthetics and strength, it is preferable for an alumina sintered body of the present invention to exhibit higher relative density. An alumina sintered body of the present invention has a relative density of, for example, preferably higher than 95%, more preferably 98% or more, even more preferably 99.5% or more. Most preferably, an alumina sintered body of the present invention is essentially void free.

In view of superior translucency and strength, an alumina sintered body of the present invention has an average crystal grain size of preferably 0.3 to 8.0 $\mu$m, more preferably 0.4 to 6.0 $\mu$m, even more preferably 0.5 to 3.0 $\mu$m. The average crystal grain size of alumina sintered body can be measured using the following method.

The alumina sintered body is photographed to capture surface images, using a scanning electron microscope (VE-9800 manufactured by Keyence under this trade name). The average crystal grain size is calculated through image analysis after indicating grain boundaries on individual crystal grains in the image. For the measurement of average crystal grain size, the captured SEM image is binarized with image analysis software (Image-Pro Plus manufactured by Hakuto Co., Ltd. under this trade name), and particles are recognized from the field (region) by adjusting the brightness range to provide clear grain boundaries. The crystal grain size from Image-Pro Plus is the average of the measurements of the length of a line segment connecting the contour line and passing through the center of gravity determined from the contour line of the crystal grain, conducted at 2-degree intervals with the center of gravity as the central point. The measurement of crystal grain size is conducted for all particles not extending beyond the edges of the SEM photographic image (3 fields) of the alumina sintered body.

The average crystal grain diameter is calculated from the crystal grain size of each particle and the number of crystal grains. The resulting arithmetic average diameter is then determined as the average crystal grain size of the sintered body. Note that particles not extending beyond the edges of the image are particles excluding those with contour lines extending beyond the screen of the SEM photographic image (particles with their contour lines interrupted by the boundary lines at the top, bottom, left, and right). To select the crystal grain size of all particles not extending beyond the edges of the image, the option in Image-Pro Plus is used that excludes all particles lying on the boundary lines.

The content of alumina and sintering aids in an alumina sintered body of the present invention is the same as that in the composition and/or pre-sintered body to be fabricated into a sintered body.

An alumina sintered body of the present invention has a translucency ($\Delta L$) of preferably 5 or more, more preferably 10 or more, even more preferably 15 or more, particularly preferably 20 or more.

Translucency ($\Delta L$) takes a lightness value L* (color space) of L*a*b* color system (JIS Z 8781-4:2013), and it is a value after subtracting a second L* value from a first L* value, where the first L* value is an L* value measured for a 1.2 mm-thick sample (sintered body) against a white background, and the second L* value is an L* value measured for the same sample against a black background.

Samples for the measurement of translucency ($\Delta L$) can be prepared as follows, for example. First, granules (composition) are press molded to provide a thickness of 1.2 mm for the sintered body to be produced, and subsequent CIP molding forms, for example, a disc-shaped molded body measuring 19 mm in diameter.

The molded body can then be fired under predetermined firing conditions, and a sintered body, or a sample, having a thickness of 1.2 mm can be prepared by polishing the surface at #2000.

For the measurement of L* values, a colorimeter (for example, CE100, analysis software Crystaleye manufactured by Olympus Corporation) can be used to measure L* values against black and white backgrounds after applying a contact liquid to a sample surface. The contact liquid may be, for example, one having a measured refractive index nD of 1.60 at 589 nm wavelength (sodium D-line).

The white background means the white part of the hiding-power test paper described in JIS K 5600-4-1:1999, Part 4, Section 1, and the black background means the black part of the hiding-power test paper.

For enhanced aesthetic appeal, it is preferable to have greater surface smoothness in an alumina sintered body of the present invention. The alumina sintered body has a surface roughness Ra of preferably 1.40 $\mu$m or less. In view of easier replication of the surface roughness of natural teeth, the surface roughness Ra is more preferably 1.25 $\mu$m or less, even more preferably 1.15 $\mu$m or less, particularly preferably 1.10 $\mu$m or less.

The alumina sintered body has a surface roughness Rz of preferably 51 $\mu$m or less. In view of easier replication of the surface roughness of natural teeth, the surface roughness Rz is more preferably 48 $\mu$m or less, even more preferably 42 $\mu$m or less, particularly preferably 39 $\mu$m or less.

The method of measurement of surface roughness Ra and Rz is as described in the EXAMPLES section below.

An alumina sintered body of the present invention may be a sintered body of a predetermined shape. For example, the sintered body may have a disc (discotic) shape, a cuboidal shape, or a shape of a dental product (for example, a crown shape).

The methods of production of the alumina compositions, granules, powders, molded bodies, pre-sintered bodies, cut or ground workpieces, and sintered bodies described in the present invention are not limited those described above, and a variety of known methods are applicable, except where specified otherwise.

The present invention encompasses embodiments combining the foregoing features in various ways within the technical idea of the present invention, provided that the present invention can exhibit its effects.

In the present invention, the upper limits and lower limits of numeric ranges (for example, ranges of contents of components, ranges of various elements (such as the average primary particle diameter), and ranges of physical properties) can be appropriately combined.

EXAMPLES

The present invention is described below in greater detail. It should be noted, however, that the present invention is in no way limited by the following Examples, and various changes may be made by a person with ordinary skill in the art within the technical idea of the present invention.

[Measurement of Relative Density]

The methods described in the Examples and Comparative Examples below were used to acquire pre-sintered bodies measuring approximately 20 mm in length, 19 mm in width, and 17 mm in height, except for using a die of a different size for the pressing of granules.

For the measurement of the relative density of pre-sintered bodies, the pre-sintered bodies were cut into 1.2 cm$^3$ samples (10.8 mm in diameter $\times$ 13 mm in height). The measurement was conducted at a pressure of 0.5 to 60,000 psia using an automatic mercury porosimeter (a pore distribution measurement device AutoPore® IV 9500 manufactured by Micromeritics, USA), in compliance with JIS R 1655:2003. The relative density was calculated from the measured porosity, using the following formula.

$$\text{(Relative density) (\%)} = \{1 - \text{(porosity)}\} \times 100$$

[Measurement of Average Primary Particle Diameter of Pre-Sintered Body]

The pre-sintered bodies obtained in the Examples and Comparative Examples below were used to prepare surface images, using a scanning electron microscope (VE-9800 manufactured by Keyence under this trade name).

For the measurement of particle diameter, the SEM image with indicated primary particles was binarized with image analysis software (Image-Pro Plus manufactured by Hakuto Co., Ltd. under this trade name), and particles were recognized from the field (region) after indicating grain boundaries on individual crystal grains in the image. In areas with unclear grain boundaries, a reduce filter was applied to the region until each region reduced to a single or multiple points, and Voronoi polygons were created by using these points as generators of Voronoi polygons. Lines were drawn that connect the midpoints between two adjacent generators, and these lines were superimposed on the original particle image to separate adjacent particles. For example, in the case where image processing produced a particle that looks like a gourd, the particle was separated into two by assuming that two adjoining circular particles are seen as a single particle.

In a processing file recognizing primary particle diameter, "Diameter" was selected from "Count/size dialog" to find the distribution (n = 4). Specifically, a mean value of particle diameters (primary particle diameters) measured in each field with the image analysis software (Image-Pro Plus) was determined for four fields from one sample.

[Measurement of Average Roundness of Pre-Sintered Body]

Using the data obtained from the measurement of average primary particle diameter, the roundness calculated by the following formula was determined as "average roundness".

$$\text{(Roundness)} = (4\pi \times \text{area}) / (\text{circumference} \times \text{circumference})$$

[Measurement of Sintering Aid Content]

The measurement was conducted using samples from the compositions, pre-sintered bodies, or sintered bodies obtained in the Examples and Comparative Examples below.

For the measurement, a field-emission scanning electron microscope (FE-SEM Reglus 8220, manufactured by Hitachi High-Tech Corporation) and an energy dispersive X-ray analyzer (Aztec Energy X-Max 50, manufactured by Oxford Instruments) were used under the following conditions (a mean value for n = 3).

Measurement magnification: 5,000×
Analysis mode: surface analysis
Acceleration voltage: 5 kV
Working distance: 15 mm ± 1 mm
X-ray extraction angle: 30 degrees
Dead time: 7%
Measurement time: 100 seconds

[Surface Polishing Method]

The alumina granules obtained in each Example and Comparative Example were filled into a die, molded by uniaxial pressing at 200 MPa, and held at 750°C for 6 hours for pre-sintering to fabricate a disc-shaped pre-sintered body measuring 14 mm in thickness and 98.5 mm in diameter.

Using three-dimensional NC data, the disc-shaped pre-sintered body was cut down to a 1 mm thick disc with an unused KATANA® drill (manufactured by Kuraray Noritake Dental Inc.; 2 mm in diameter, non-diamond coated), using a milling machine DWX-52DC manufactured by Kuraray Noritake Dental Inc.

The machining was conducted in contour patterns with a spindle rotational speed of 30,000 rpm, a feed rate of 2,000 mm/min, a machining pitch of Z = 0.5 mm, and XY = 1 mm, outwardly from the center of the disc-shaped pre-sintered body. Using the Noritake Pro-Tec Diamond Point DP-04 manufactured by Kuraray Noritake Dental Inc., the pillar was separated from the thin disc of 1 mm thickness prepared by machining. On the surface of the thin disc, steps originating from CAD/CAM processing were observed.

Using a polisher (KATANA® Zirconia TWIST DIA MEDIUM) manufactured by Kuraray Noritake Dental Inc., the entire surface of the thin disc was coarsely polished for 5 minutes at a rotational speed of 4,000 rpm and a load of 0.05 kgf.

This was followed by final polishing, which was conducted over the entire surface for 5 minutes at a rotational speed of 4,000 rpm and a load of 0.01 kgf, using a different polisher (KATANA® Zirconia TWIST DIA FINE) manufactured by Kuraray Noritake Dental Inc.

For finishing, the entire surface underwent additional polishing for 5 minutes at a rotational speed of 4,000 rpm and a load of 0.005 kgf, using the felt wheel included in the abrasive (Pearl Surface®) manufactured by Kuraray Noritake Dental Inc.

Lastly, any remaining polishing powder was brushed away using a size 10 Kolinsky-Tajmyr Sable brush manufactured by ESCODA.

[Measurement of Surface Roughness]

The pre-sintered bodies or sintered bodies obtained in Examples and Comparative Examples underwent nine measurements with a stylus-type surface roughness meter (DekTak-150 manufactured by Bruker Japan) under the following conditions.

Measurement distance: 10 mm
Scan Length: 15,000 $\mu$m
Scan Duration: 100 see
Measurement Range: 65.5 $\mu$m
Stylus Force: 1.00 mg
The results obtained were expressed as Zn(X) Xn = 0.0005 [mm] $\times$ n (n = 0, 1, 2, ..., 9)

From these results, a third-order fitting function (Z'n(X) = aX + b) was determined using the least squares method. The tilt of the sample during measurement was eliminated by taking the difference between the measurement results and the fitting function.

Additionally, the arithmetic mean roughness Ra was determined using the following formula based on the aforementioned differences. The maximum height roughness Rz was determined as the maximum value of Zn(X).

[Math. 1]

$$Ra = \frac{1}{N}\sum_{n=1}^{N} |Z_n(X_n) - Z'_n(X_n)|$$

The measurement results for the surface roughness of pre-sintered bodies before polishing by the surface polishing method described above are presented in Table 2 under "Before polishing", whereas the measurement results for the surface roughness of pre-sintered bodies polished by the surface polishing method are presented in Table 2 under "After polishing"

Similarly, the measurement results for the surface roughness of sintered bodies with no polishing of the pre-sintered bodies are presented in Table 3 under "No polishing as pre-sintered body", whereas the measurement results for the surface roughness of sintered bodies involving polishing of the pre-sintered bodies are presented in Table 3 under "Polished as pre-sintered body".

[Strength Measurement of Pre-Sintered Body]

Measurement was conducted using disc-shaped pre-sintered bodies measuring 14 mm in thickness and 98.5 mm in diameter, similar to the samples employed in the surface polishing method. The disc-shaped pre-sintered bodies were cut into samples (5 mm $\times$ 10 mm $\times$ 50 mm) in compliance with ISO 6872:2015. For surface finishing, the sample surfaces, including chamfered surfaces (45° chamfers at the corners of sample; see ISO 6872:2015, Section 7.3.1.2.1), were finished longitudinally with #600 sandpaper.

The sample was disposed in such an orientation that its widest face was perpendicular to the vertical direction (loading direction), and was measured for three-point flexural strength at a span (the distance between supports) of 30 mm and a crosshead speed of 0.5 mm/min, using a universal testing machine (AG-I 100kN manufactured by Shimadzu Corporation) (a mean value for n = 3).

[Hardness Evaluation]

The pre-sintered bodies obtained in the Examples and Comparative Examples below were cut into crown shapes using a CAD/CAM system. The cut workpiece, integrated with the pillar (cylindrical in shape with a diameter of 5 mm) forming a part of the pre-sintered body, was evaluated according to the evaluation criteria below after being machined with an ULTIMATE 500 (manufactured by Nakanishi Inc.) fitted with a Diamond Point HP, shape number 25 (manufactured by Shofu Inc.). For machining, the Diamond Point HP was pressed against the pillar portion, perpendicular to the lengthwise direction of the pillar, applying a load of about 500 g at 10,000 rpm in the air while visually monitoring the process (n = 10).

The pre-sintered bodies were determined as "Good" when at least 8 out of the 10 samples had a score of A, and 0 to 2 samples had a score of B, "Moderate" when at least 8 out of the 10 samples had a score of A, and 1 or 2 samples had a score of C, "Poor" when 3 or more samples had a score of B, and "Very poor" when 3 or more samples had a score of C.

Samples with a score of C are more likely to detach from the pillar and become unusable, whereas those with a B score might become usable with an extended machining time. Hence, a rating of "good" is preferable over "Moderate".

<Evaluation Criteria>

A: At least 90% of the pillar was cut within 10 seconds
B: The pillar was not cuttable within 10 seconds (too hard)
C: The pillar broke before at least 90% of the pillar was cut within 10 seconds (too soft)

[Production of Pre-Sintered Body]

<Examples 1 and 7>

After weighing 1,000 g of $\alpha$-alumina raw material NXA-100 (average primary particle diameter: 100 nm; manufactured by Sumitomo Chemical Co., Ltd.) and an equivalent to 0.1 g of magnesium chloride, these were introduced into 10 L of ethanol and ultrasonically dispersed.

These were introduced into a rotary container along with alumina beads, and the alumina raw material, including aggregated particles, was pulverized with a ball mill until the desired average primary particle diameter was achieved through mixing and crushing. The primary particle diameter was measured by volume with ultrasonic waves being applied after a slurry diluted with ethanol was subjected to 30 minutes of ultrasonication, using a laser diffraction/scattering particle size distribution analyzer (manufactured by Horiba Ltd. under the trade name Partica LA-950). The desired slurry with hardly any secondary aggregation was obtained after about 1 hour of ball milling.

The slurry was divided into separate 2 L beakers, and stirred with rotary vanes at 200 rpm for 1 hour. Following an abrupt cessation of rotary vanes movement, the slurry was left to stand for 15 minutes. Settling of white particles was visible at the bottom of the beaker, and the supernatant appeared cloudy. The upper third of the slurry within the beaker was extracted as the slurry for Example 1, while the lower third of the slurry within the beaker was used as the slurry for Example 7. In Table 1, the distinction between slurries is based on elutriation, with Example 1 labeled as "Above NXA-100 elutriation", and Example 7 designated as "Below NXA-100 elutriation".

Subsequently, an organic binder was added to the slurry. An aqueous acrylic binder, serving as the organic binder, was added in an amount of 2.5 mass% with respect to the $\alpha$-alumina raw material (the organic binder content relative to the entire slurry). The mixture underwent stirring with rotary vanes for 24 hours. After stirring, the slurries underwent drying and granulation with a spray dryer, yielding alumina granules. The granules had an average particle diameter of 40 $\mu$m. An amount of 350 g of the powder formed by these granules was introduced into a cylindrical die measuring 100 mm in diameter, and uniaxially pressed at 150 MPa to yield a molded body. The molded body was placed in an electric furnace, and heated from room temperature at 3°C/min. Following a 2-hour retention at 500°C for the purpose of debinding organic components, the molded body was maintained for 6 hours at the highest pre-sintering temperature presented in Table 2, and gradually cooled at -0.4°C/min from the highest pre-sintering temperature to yield pre-sintered bodies.

<Examples 2 to 5, Comparative Example 7>

After weighing 100 g of $\alpha$-alumina raw material NXA-100 (average primary particle diameter: 100 nm; manufactured by Sumitomo Chemical Co., Ltd.) or NXA-150 (average primary particle diameter: 150 nm; manufactured by Sumitomo Chemical Co., Ltd.) and an equivalent to 0.1 g of magnesium chloride, these were introduced into 1 L of ethanol and ultrasonically dispersed.

These were introduced into a rotary container along with alumina beads, and the raw material was pulverized with a ball mill until the desired primary particle diameter was achieved through mixing and crushing. The primary particle diameter was measured by volume with ultrasonic waves being applied after a slurry diluted with ethanol was subjected to 30 minutes of ultrasonication, using a laser diffraction/scattering particle size distribution analyzer (manufactured by Horiba Ltd. under the trade name Partica LA-950). The desired slurry with hardly any secondary aggregation was obtained after about 1 hour of ball milling.

Subsequently, an organic binder was added to the slurry. An aqueous acrylic binder, serving as the organic binder, was added in an amount of 2.5 mass% with respect to the $\alpha$-alumina raw material (the organic binder content relative to the entire slurry). The mixture underwent stirring with rotary vanes for 24 hours.

After stirring, the slurry underwent drying and granulation with a spray dryer to yield granules. The granules had an average particle diameter of 40 $\mu$m. An amount of 350 g of the powder formed by these granules was introduced into a cylindrical die measuring 100 mm in diameter, and uniaxially pressed at 150 MPa to yield a molded body. The molded body was placed in an electric furnace, and heated from room temperature at 3°C/min. Following a 2-hour retention at 500°C for the purpose of debinding organic components, the molded body was heated to the temperature presented in Table 2 at 3°C/min and maintained for 6 hours at it as the highest pre-sintering temperature, and gradually cooled at -0.4°C/min from this pre-sintering temperature to yield pre-sintered bodies.

<Examples 6, 8, and 9, Comparative Examples 3 to 5, and 8>

The same procedure employed in Example 2 was used to obtain pre-sintered bodies, with the exception that AKP-53, AA-03, AA-05, and AA-07 (manufactured by Sumitomo Chemical Co., Ltd.) were used as $\alpha$-alumina raw materials, and that the highest pre-sintering temperature in pre-sintering was varied as shown in Table 2.

<Comparative Examples 1 and 2>

The same procedure employed in Example 2 was used to obtain pre-sintered bodies, with the exception that an equivalent to 0.12 g of magnesium chloride was used relative to 100 g of $\alpha$-alumina raw material, and that the highest pre-sintering temperature in pre-sintering was varied as shown in Table 2.

<Comparative Example 6>

The same procedure employed in Example 2 was used to obtain pre-sintered bodies, with the exception that uniaxial pressing was carried out at a pressure of 15 MPa.

<Examples 10 to 13>

The same procedure employed in Example 3 was used to obtain pre-sintered bodies, with the exception that the type and amount of sintering aid were varied as shown in Table 1.

[Production of Sintered Body]

The pre-sintered body produced in each Example and Comparative Example was cut into a thin disc shape. After surface polishing, the sample was heated from room temperature at 3°C/min to the highest sintering temperature presented in Table 3 in an atmospheric environment, using the NORITAKE KATANA System KATANA® F-1N (manufactured by Kuraray Noritake Dental Inc.). Following a 2-hour retention at this highest sintering temperature, the sample was gradually cooled at -0.4°C/min from the highest sintering temperature to yield a sintered body

The results for Examples and Comparative Examples are presented in Table 3.

[Table 1]

| | | Composition | | | | | |
|---|---|---|---|---|---|---|---|
| | | Sintering aid[1)] | | | | | |
| | Main ingredient | Sintering aid 1 | | Sintering aid 2 | | Sintering aid 3 | |
| | | Type | Amount [ppm] | Type | Amount [ppm] | Type | Amount [ppm] |
| Ex. 1 | Above NXA-100 elutriation | $MgCl_2$ | 1000 | - | - | - | - |
| Ex. 2 | NXA-100 | $MgCl_2$ | 1000 | - | - | - | - |
| Ex. 3 | NXA-100 | $MgCl_2$ | 1000 | - | - | - | - |
| Ex. 4 | NXA-150 | $MgCl_2$ | 1000 | - | - | - | - |
| Ex. 5 | NXA-150 | $MgCl_2$ | 1000 | - | - | - | - |
| Ex. 6 | AKP-53 | $MgCl_2$ | 1000 | - | - | - | - |
| Ex. 7 | Below NXA-100 elutriation | $MgCl_2$ | 1000 | - | - | - | - |
| Ex. 8 | AA-03 | $MgCl_2$ | 1000 | - | - | - | - |
| Ex. 9 | AA-05 | $MgCl_2$ | 1000 | - | - | - | - |
| Ex. 10 | NXA-100 | $Mg(OH)_2$ | 100 | - | - | - | - |
| Ex. 11 | NXA-100 | - | - | $ZrO_2$ | 100 | - | - |
| Ex. 12 | NXA-100 | - | - | $ZrO_2$ | 500 | $Y_2O_3$ | 57 |
| Ex. 13 | NXA-100 | $MgCl_2$ | 1000 | $ZrO_2$ | 1000 | $Y_2O_3$ | 113 |
| Com. Ex. 1 | NXA-100 | $MgCl_2$ | 1200 | | | | |
| Com. Ex. 2 | NXA-100 | $MgCl_2$ | 1200 | | | | |
| Com. Ex. 3 | AA-03 | $MgCl_2$ | 1000 | | | | |
| Com. Ex. 4 | AA-05 | $MgCl_2$ | 1000 | | | | |
| Com. Ex. 5 | AA-07 | $MgCl_2$ | 1000 | | | | |
| Com. Ex. 6 | NXA-100 | $MgCl_2$ | 1000 | | | | |
| Com. Ex. 7 | NXA-100 | $MgCl_2$ | 1000 | | | | |
| Com. Ex. 8 | AKP-53 | $MgCl_2$ | 1000 | | | | |
| 1) The amount of sintering aid represents a value in terms of an element (for example, Mg equivalent when the sintering aid is $MgCl_2$.) | | | | | | | |

[Table 2]

| | Production conditions | | Pre-sintered body | | | | | | | | |
| | Applied pressure [MPa] | Pre-sintering temperature[1] [°C] | Average roundness | Relative density [%] | Average primary particle diameter [nm] | Strength [MPa] | Before polishing | | After polishing | | Hardness |
| | | | | | | | Surface roughness Ra [μm] | Surface roughness Rz [μm] | Surface roughness Ra [μm] | Surface roughness Rz [μm] | |
|---|---|---|---|---|---|---|---|---|---|---|---|
| Ex. 1 | 150 | 600 | 0.86 | 50 | 40 | 25 | 9.9 | 91.2 | 0.91 | 31.7 | Good |
| Ex. 2 | 150 | 750 | 0.83 | 51 | 95 | 23 | 12.1 | 81.3 | 1.11 | 28.2 | Good |
| Ex. 3 | 150 | 800 | 0.83 | 51 | 100 | 25 | 14.3 | 87.4 | 1.30 | 30.3 | Good |
| Ex. 4 | 150 | 800 | 0.82 | 52 | 150 | 13 | 13.5 | 94.7 | 1.24 | 32.9 | Moderate |
| Ex. 5 | 150 | 1000 | 0.83 | 52 | 150 | 38 | 16.2 | 108.4 | 1.46 | 37.8 | Good |
| Ex. 6 | 150 | 1050 | 0.84 | 49 | 170 | 35 | 12.1 | 140.5 | 1.15 | 49.4 | Good |
| Ex. 7 | 150 | 1000 | 0.86 | 53 | 200 | 45 | 14.3 | 126.3 | 1.36 | 44.7 | Good |
| Ex. 8 | 150 | 1150 | 0.81 | 52 | 410 | 15 | 16.5 | 150.6 | 1.55 | 52.6 | Moderate |
| Ex. 9 | 150 | 1150 | 0.83 | 52 | 580 | 16 | 17.1 | 151.2 | 1.64 | 52.8 | Moderate |
| Ex. 10 | 150 | 800 | 0.83 | 51 | 100 | 25 | 10.2 | 98.1 | 1.02 | 32.1 | Good |
| Ex. 11 | 150 | 800 | 0.82 | 52 | 100 | 28 | 11.8 | 101.2 | 1.11 | 35.7 | Good |
| Ex. 12 | 150 | 800 | 0.82 | 51 | 100 | 29 | 12.7 | 112.3 | 1.23 | 39.4 | Good |
| Ex. 13 | 150 | 800 | 0.82 | 51 | 100 | 32 | 14.3 | 122.1 | 1.36 | 45.6 | Good |
| Com. Ex. 1 | 150 | 1200 | 0.77 | 56 | 97 | 45 | 21.2 | 158.4 | 1.76 | 54.2 | Good |
| Com. Ex. 2 | 150 | 1250 | 0.76 | 58 | 98 | 58 | 24.4 | 168.9 | 1.97 | 58.0 | Poor |
| Com. Ex. 3 | 150 | 1000 | 0.79 | 55 | 400 | 9 | 26.8 | 175.3 | 2.24 | 60.3 | Very poor |
| Com. Ex. 4 | 150 | 1000 | 0.77 | 52 | 580 | 9 | 30.6 | 210.8 | 2.60 | 72.5 | Very poor |
| Com. Ex. 5 | 150 | 1200 | 0.75 | 41 | 880 | 11 | 35.2 | 202.7 | 3.01 | 69.6 | Moderate |

| | Production conditions | | Pre-sintered body | | | | | | | | |
|---|---|---|---|---|---|---|---|---|---|---|---|
| | Applied pressure [MPa] | Pre-sintering temperature[1] [°C] | Average roundness | Relative density [%] | Average primary particle diameter [nm] | Strength [MPa] | Before polishing | | After polishing | | Hardness |
| | | | | | | | Surface roughness Ra [μm] | Surface roughness Rz [μm] | Surface roughness Ra [μm] | Surface roughness Rz [μm] | |
| Com. Ex. 6 | 15 | 800 | 0.83 | 42 | 100 | 9 | 24.6 | 170.2 | 2.06 | 60.1 | Very poor |
| Com. Ex. 7 | 700 | 800 | 0.84 | 65 | 105 | 25 | 22.9 | 162.1 | 1.81 | 55.1 | Poor |
| Com. Ex. 8 | 150 | 800 | 0.85 | 42 | 170 | 7 | 28.9 | 185.5 | 2.31 | 64.8 | Very poor |
| 1) Highest pre-sintering temperature | | | | | | | | | | | |

[Table 3]

| | Highest sintering temperature [°C] | Sintered body | | | |
|---|---|---|---|---|---|
| | | No polishing as pre-sintered body | | Polished as pre-sintered body | |
| | | Surface roughness Ra [$\mu$m] | Surface roughness Rz [Nm] | Surface roughness Ra [$\mu$m] | Surface roughness Rz [$\mu$m] |
| Ex. 1 | 1375 | 7.0 | 42.9 | 0.69 | 23.7 |
| Ex. 2 | 1400 | 10.1 | 67.8 | 0.98 | 37.3 |
| Ex. 3 | 1400 | 10.6 | 69.0 | 1.04 | 38.1 |
| Ex. 4 | 1400 | 11.5 | 64.7 | 1.13 | 35.7 |
| Ex. 5 | 1400 | 14.1 | 67.0 | 1.39 | 37.0 |
| Ex. 6 | 1400 | 11.2 | 83.9 | 1.07 | 46.3 |
| Ex. 7 | 1400 | 10.5 | 76.8 | 1.01 | 42.2 |
| Ex. 8 | 1450 | 11.9 | 91.1 | 1.15 | 50.1 |
| Ex. 9 | 1450 | 12.4 | 91.0 | 1.22 | 50.2 |
| Ex. 10 | 1400 | 7.2 | 48.9 | 0.78 | 25.1 |
| Ex. 11 | 1400 | 10.2 | 70.1 | 0.99 | 39.4 |
| Ex. 12 | 1400 | 11.8 | 71.8 | 1.04 | 40.2 |
| Ex. 13 | 1400 | 12.8 | 73.9 | 1.18 | 42.1 |
| Com. Ex. 1 | 1400 | 17.1 | 95.2 | 1.46 | 51.2 |
| Com. Ex. 2 | 1400 | 22.2 | 101.9 | 1.96 | 54.6 |
| Com. Ex. 3 | 1450 | 23.9 | 109.8 | 2.10 | 58.2 |
| Com. Ex. 4 | 1450 | 23.0 | 109.9 | 2.03 | 58.8 |
| Com. Ex. 5 | 1450 | 29.7 | 121.1 | 2.69 | 66.1 |
| Com. Ex. 6 | 1400 | 23.5 | 108.2 | 2.01 | 58.5 |
| Com. Ex. 7 | 1400 | 18.9 | 97.7 | 1.78 | 53.4 |
| Com. Ex. 8 | 1400 | 27.1 | 111.1 | 2.21 | 60.1 |

INDUSTRIAL APPLICABILITY

An oxide ceramic pre-sintered body for dental use of the present invention can be suitably used for machining such as CAD/CAM.

**Claims**

1. An oxide ceramic pre-sintered body for dental use comprising oxide ceramic particles with an average roundness of primary particles of 0.81 or more and having a relative density of 43 to 63%.

2. The oxide ceramic pre-sintered body for dental use according to claim 1, wherein the particles have an average primary particle diameter of 30 to 600 nm.

3. The oxide ceramic pre-sintered body for dental use according to claim 1 or 2, which has a three-point flexural strength of 10 to 50 MPa.

4. The oxide ceramic pre-sintered body for dental use according to any one of claims 1 to 3, which has a surface roughness Ra of 1.70 $\mu$m or less after polishing.

5. The oxide ceramic pre-sintered body for dental use according to any one of claims 1 to 4, which has a surface roughness Rz of 54 $\mu$m or less after polishing.

6. The oxide ceramic pre-sintered body for dental use according to any one of claims 1 to 5, wherein the oxide ceramic particles comprise zirconia and/or alumina.

7. The oxide ceramic pre-sintered body for dental use according to claim 6, wherein the alumina comprises $\alpha$-alumina particles with a purity of 99.5% or more.

8. The oxide ceramic pre-sintered body for dental use according to claim 6 or 7, which further comprises a sintering aid, and the sintering aid comprises at least one element selected from the group consisting of a Group 2 element, Ce, Zr, and Y

9. The oxide ceramic pre-sintered body for dental use according to any one of claims 1 to 8, which has a surface roughness Ra of 1.40 $\mu$m or less after being fired into a sintered body under atmospheric pressure without a hot isostatic pressing process.

10. The oxide ceramic pre-sintered body for dental use according to any one of claims 1 to 9, which has a surface roughness Rz of 51 $\mu$m or less after being fired into a sintered body under atmospheric pressure without a hot isostatic pressing process.

11. A method for producing an oxide ceramic pre-sintered body for dental use, comprising the steps of:

   press molding an oxide ceramic composition at a surface pressure of 20 to 600 MPa; and
   firing the resultant molded body at 400°C or more and less than 1,200°C under atmospheric pressure,
   the oxide ceramic pre-sintered body for dental use comprising oxide ceramic particles with an average roundness of primary particles of 0.81 or more and having a relative density of 43 to 63%.

12. The method for producing an oxide ceramic pre-sintered body for dental use according to claim 11, wherein the oxide ceramic particles comprise zirconia and/or alumina.

13. The method for producing an oxide ceramic pre-sintered body for dental use according to claim 12, wherein the alumina comprises $\alpha$-alumina particles with a purity of 99.5% or more.

14. The method for producing an oxide ceramic pre-sintered body for dental use according to any one of claims 11 to 13, wherein the oxide ceramic pre-sintered body for dental use further comprises a sintering aid, and the sintering aid comprises at least one element selected from the group consisting of a Group 2 element, Ce, Zr, and Y

15. A method for producing an oxide ceramic sintered body for dental use, comprising the step of polishing an oxide ceramic pre-sintered body for dental use of any one of claims 1 to 10 with dental polishing equipment.

16. The method for producing an oxide ceramic sintered body for dental use according to claim 15, which comprises the step of sintering the oxide ceramic pre-sintered body for dental use under atmospheric pressure without a hot isostatic pressing process.

FIG.1

## INTERNATIONAL SEARCH REPORT

International application No.

**PCT/JP2022/046484**

### A.  CLASSIFICATION OF SUBJECT MATTER

*A61K 6/802*(2020.01)i; *A61C 5/77*(2017.01)i; *A61C 13/083*(2006.01)i; *A61K 6/818*(2020.01)i; *C04B 35/111*(2006.01)i;
*C04B 35/486*(2006.01)i
FI:   A61K6/802; A61C5/77; A61C13/083; A61K6/818; C04B35/111; C04B35/486

According to International Patent Classification (IPC) or to both national classification and IPC

### B.  FIELDS SEARCHED

Minimum documentation searched (classification system followed by classification symbols)

A61K6/802; A61C5/77; A61C13/083; A61K6/818; C04B35/111; C04B35/486

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C.  DOCUMENTS CONSIDERED TO BE RELEVANT

| Category* | Citation of document, with indication, where appropriate, of the relevant passages | Relevant to claim No. |
|---|---|---|
| X | WO 2021/100876 A1 (KURARAY NORITAKE DENTAL INC.) 27 May 2021 (2021-05-27) claims, paragraphs [0006]-[0010], [0025]-[0033], [0037], [0040], [0041], [0048]-[0076], examples | 1-16 |
| X | WO 2018/56331 A1 (KURARAY NORITAKE DENTAL INC.) 29 March 2018 (2018-03-29) claims, paragraphs [0050]-[0052], [0057]-[0085], [0099]-[0122], [0135], examples | 1-16 |
| A | JP 2010-220779 A (NORITAKE CO., LIMITED) 07 October 2010 (2010-10-07) paragraphs [0031], [0039], [0040] | 1-16 |
| A | JP 2020-142983 A (TOSOH CORP.) 10 September 2020 (2020-09-10) paragraph [0092] | 1-16 |
| A | JP 2013-119485 A (PILOT CORP.) 17 June 2013 (2013-06-17) entire text | 1-16 |

☐ Further documents are listed in the continuation of Box C.   ☑ See patent family annex.

| | |
|---|---|
| *   Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **01 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/046484**

| Patent document cited in search report | | | Publication date (day/month/year) | Patent family member(s) | | | Publication date (day/month/year) |
|---|---|---|---|---|---|---|---|
| WO | 2021/100876 | A1 | 27 May 2021 | CN | 114650967 | A | |
| WO | 2018/56331 | A1 | 29 March 2018 | US | 2019/0231651 | A1 | |
| | | | | claims, paragraphs [0055]-[0057], [0062]-[0088], [0102]-[0125], [0138], examples | | | |
| | | | | EP | 3517503 | A4 | |
| | | | | CN | 109790047 | A | |
| | | | | KR | 10-2019-0047702 | A | |
| JP | 2010-220779 | A | 07 October 2010 | US | 2010/0248936 | A1 | |
| | | | | paragraphs [0036], [0044] | | | |
| JP | 2020-142983 | A | 10 September 2020 | US | 2020/0283343 | A1 | |
| | | | | paragraph [0186] | | | |
| | | | | EP | 3705291 | A1 | |
| JP | 2013-119485 | A | 17 June 2013 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015037537 A
- JP 2012180275 A
- WO 2009045940 A